# EUROPEAN PATENT APPLICATION

(11) **EP 1 369 480 A1**
(43) Date of publication of application: **10.12.2003**
(21) Application number: 02253911.8
(22) Date of filing: 05.06.2002
(51) Int. Cl.: C12N 15/10, C12Q 1/68

(54) **Process for preparation of full-length cDNA and anchor used for the same**

(71) Applicant: Bioneer Corporation, Daejon 306-220 (KR)
(72) Inventor: Park, Han-Oh, c/o Bioneer Corporation, Daejon 306-220 (KR); Jeon, Jin-Tae, c/o Bioneer Corporation, Daejon 306-220 (KR); Jang, Mi-Sook, c/o Bioneeer Corporation, Daejon 306-220 (KR)
(74) Representative: Owen, Deborah Jane

(57) **Abstract**

The present invention relates to a process for the preparation of full-length complementary DNA(cDNA). More particularly, the present invention is directed to a process for selective amplification of full-length cDNA, which comprises: i) a step for preparing a hybrid composed of a messenger RNA(mRNA) strand and a cDNA strand of which three(3) or four(4) deoxycitidinemono phosphate(dCMP) are combined at the 3' end, by treating mRNA with reverse transcriptase; separately from the above step, ii) a step for adenylating a single strand anchor of which a biotin or phosphate group is combined at the 3' end, and a phosphate group is combined at the 5' end; iii) a step for ligating said adenylated single strand anchor to the 3' end of the full-length cDNA strand of said cDNA/mRNA hybrid to select the full-length cDNA/mRNA hybrid; and iv) a step for amplifying only the full-length cDNA/mRNA hybrid through polymerase chain reaction(PCR) which employs a primer of which base sequence is complementary to that of said anchor.

SEQUENCE LISTING

<110> Bioneer Corporation

<120> Process for preparation of full-length cDNA and anchor used
 for the same

<130> 17.14.78459

<160> 12

<170> PatentIn version 3.0

<210> 1
<211> 31
<212> DNA
<213> Artificial/Unknown

<220>
<221> misc_feature
<222> ()..()
<223> Anchor sequence, 5' phosphate and 3' biotin


<400> 1
aagcagtggt atcaacgagt gcggccgcgg g
    31


<210> 2
<211> 54
<212> DNA
<213> Artificial/Unknown

<220>
<221> misc_feature
<222> ()..()
<223> Primer


<400> 2
attctagagc ggccgcgaca tgtttttttt tttttttttt tttttttttt ttvn
    54


<210> 3
<211> 34
<212> DNA
<213> Artificial/Unknown

<220>
<221> misc_feature
<222> ()..()

<223>  Primer

<400>  3
cccgcggccg cactcgttga taccactgct tggg
    34

<210>  4
<211>  35
<212>  DNA
<213>  Artificial/Unknown

<220>
<221>  misc_feature
<222>  ()..()
<223>  Primer

<400>  4
cccgcggccg cactcgttga taccactgct tgggg
    35

<210>  5
<211>  26
<212>  DNA
<213>  Artificial/Unknown

<220>
<221>  misc_feature
<222>  ()..()
<223>  Primer

<400>  5
cgcactcgtt gataccactg cttggg
    26

<210>  6
<211>  27
<212>  DNA
<213>  Artificial/Unknown

<220>
<221>  misc_feature
<222>  ()..()
<223>  Primer

<400>  6
cgcactcgtt gataccactg cttgggg

27

<210> 7
<211> 30
<212> DNA
<213> Artificial/Unknown

<220>
<221> misc_feature
<222> ()..()
<223> Primer

<400> 7
gccctgaggc actcttccag ccttccttcc
30

<210> 8
<211> 30
<212> DNA
<213> Artificial/Unknown

<220>
<221> misc_feature
<222> ()..()
<223> Primer

<400> 8
gtcatactcc tgcttgctga tccacatctg
30

<210> 9
<211> 30
<212> DNA
<213> Artificial/Unknown

<220>
<221> misc_feature
<222> ()..()
<223> Primer

<400> 9
gtggcgtata gtaaggctgc aacagttact
30

<210> 10
<211> 30

<212>  DNA
<213>  Artificial/Unknown

<220>
<221>  misc_feature
<222>  ()..()
<223>  Primer


<400>  10
aagcagttgg tggtgcagga ggcattgctg
    30


<210>  11
<211>  30
<212>  DNA
<213>  Artificial/Unknown

<220>
<221>  misc_feature
<222>  ()..()
<223>  Primer


<400>  11
cactgatgag gtcggtgatg gcgttggtaa
    30


<210>  12
<211>  30
<212>  DNA
<213>  Artificial/Unknown

<220>
<221>  misc_feature
<222>  ()..()
<223>  Primer


<400>  12
tgctggtacc aagaaccgct ttatccagat
    30

## Description

The present invention relates to a process for the preparation of full-length complementary DNA(cDNA). More particularly, the present invention is directed to a process for selective amplification of full-length cDNA, which comprises: i) a step for preparing a hybrid composed of a messenger RNA(mRNA) strand and a cDNA strand of which three(3) or four(4) deoxycitidinemono phosphate(dCMP) are combined at the 3' end, by treating the mRNA with reverse transcriptase; separately from the above step, ii) a step for adenylating a single strand anchor of which a biotin or phosphate group is combined at the 3' end, and a phosphate group is combined at the 5' end; iii) a step for ligating said adenylated single strand anchor to the 3' end of the full-length cDNA strand of said cDNA/mRNA hybrid to select full-length cDNA/mRNA hybrid; and iv) a step for amplifying only the full-length cDNA/mRNA hybrid through a polymerase chain reaction(PCR) which employs a primer which base sequence is complementary to that of said anchor.

Recently, new techniques for mass production of various genetic engineering products such as proteins, have been developed through identification of novel genes and determination of base sequences thereof, and then characterization of their biological properties.

In addition, some methods for the treatment of various diseases caused by inappropriate expression and/or suppression of a specific gene or by the influence of a foreign substance such as a carcinogen or teratogen, can be developed through the analysis of the base sequences of the gene.

To these ends, a process for the mass-production of protein having significant biological application, wherein cDNA prepared from mRNA through reverse transcription is inserted into a cloning vector to be cloned, have been developed as a basic skill in biotechnology.

Therefore, the development of more efficient and simple processes for the production of full-length cDNA on a large scale, have been needed for the mass-production of human cDNA libraries and for the study of gene expression patterns by using DNA chips.

As a prerequisite tool for the research of the expression pattern of human genes and the structure of protein prepared therefrom, the method of 5', 3' end rapid amplification of cDNA end(RACE) is used widely, and the method for determination of complete base sequence by combining those of expressed sequence tags (ESTs) obtained by partial amplification of cDNA, has been developed.

At present a the method for the preparation of full-length cDNA wherein the cap structure of mRNA is used as an identification marker of full-length cDNA, has been developed. The cap structure of mRNA is a characteristic part of the 5' end of eukaryotic mRNA, and contains guanidine nucleotide substituted with a methyl group. In general, the cap structure of mRNA is the site recognized by initiation factors in protein synthesis processes.

At present, several methods for recognizing the cap structure of the eukaryotic mRNA, for example, a method wherein a fusion protein composed of a cap binding protein and a solid support matrix, is bound on the 5' cap site; a method wherein biotin which can recognize a diol group of the cap structure is employed; or a method wherein the 5'cap structure of mRNA is removed by using tobacco acid pyrophosphatase and then synthetic oligonucleotide is ligated thereto, etc., are used frequently.

However, these methods are not cost-efficient and moreover, these methods are time-consuming because the enzyme treatment step and the purification step requires a long time and are so complicated that starting materials may be decomposed or lost during these steps.

Therefore, recently, other methods, e.g. the so called "CapFinder" method or "CapSelect" method have been used as typical methods for identification of the cap structure of the 5' end of the eukaryotic mRNA during the reverse transcription. However, it takes a long time to add nucleotide on the cap structure of mRNA by employing reverse transcriptase in the CapFinder method, and in addition, the incomplete amplification pattern may occur since the reverse transcriptase has to recognize template switching oligonucleotide.

The "Capselect" method overcomes partly the above problems. However, the "Capselect" method also has drawbacks in that it requires an additional step wherein an adenine group is added through a Ribotailing step by using terminal deoxyribonucleotidyl tranferase, and that it requires a step wherein double strand adaptor is linked again. Therefore, the "Capselect" method is inappropriate to be employed as a commercial method for the production of full-length cDNA in large scale since it needs considerable time, at least more than ten hours to link the doublestranded anchor.

Therefore, a novel process by which full-length cDNA can be obtained through more efficient and simple procedures than before, and by which full-length cDNA can be amplified completely to produce full-length cDNA in large scale, has been awaited in this field.

The purpose of the present invention, therefore, is to provide a novel process to produce full-length cDNA in large scale, comprising a step for obtaining full-length cDNA through only two procedures, reverse transcription of mRNA to obtain cDNA and ligation of anchor and nucleic acid; and a step for amplifying completely the full-length cDNA thus obtained.

### Brief description of the drawings

The above objects and other advantages of the present invention will become more apparent by describing in detail the examples thereof with reference to the attached drawings, in which:
Fig.1 represents the result of agarose gel electrophoresis of the products obtained by amplification of both ends of β-actin cDNA by the process and the primer of the present invention.
Fig. 2 represents the result of agarose gel electrophoresis of the products obtained by the amplification of the 5' end of full-length GAPDH(Lane1), full-length β-actin(Lane2), full-length RNA polymerase II(Lane3) and full-length TFR(Lane4) prepared from 100ng of mRNA by using the process and primer of the present invention.
Fig. 3 represents the result of agarose gel electrophoresis of the products obtained by the amplification of GAPDH(Lane1), γ-actin(Lane 2), RNA polymerase II(Lane3) prepared from 2µg of total spleen mRNA by using the process and the primer of the present invention.
Fig. 4 represents the result of analysis wherein the base sequences determined by amplifying the 5' end of full-length GAPDH(Fig. 4-1), full-length TFR(Fig. 4-2), and full-length RNA polymerase II(Fig. 4-3) by using the process and the primer of the present invention, and then cloning them, are compared with the full-length sequences which have been reported previously.
Fig. 5 represents schematically various steps in the processes of the present invention for preparing full-length cDNA.

### Disclosure of invention

The object of the present invention is to provide a process for selective amplification of full-length cDNA or mRNA. More particularly, the object of the present invention is to provide a process for selective amplification of full-length cDNA or mRNA, which comprises: i) a step for preparing a hybrid composed of an mRNA strand and a cDNA strand of which three(3) or four(4) dCMPs are combined, incorporated or attached at the 3' end, by treating mRNA with reverse transcriptase; separately from the above step, ii) a step for adenylating a single strand anchor of which a biotin or phosphate group is combined, incorporated or attached at the 3' end and a phosphate group is combined, incorporated or attached at the 5' end; iii) a step for ligating said adenylated single strand anchor selectively to the 3' end of the full-length cDNA strand of said cDNA/mRNA hybrid to select full-length cDNA/mRNA hybrid; and iv) a step for amplifying only the full-length cDNA strand through a polymerase chain reaction (PCR) which employs a primer of which base sequence is complementary to that of said anchor.

Another object of the present invention is to provide a process for selective amplification of a specific part of a cDNA or mRNA, which comprises: i) a step for preparing a hybrid composed of an mRNA strand and a cDNA strand of which three(3) or four(4) dCMPs are combined, incorporated or attached at the 3' end, by treating mRNA with reverse transcriptase; separately from the above step, ii) a step for adenylating a single strand anchor of which a biotin or phosphate group is combined, incorporated or attached at the 3' end and a phosphate group is combined, incorporated or attached at the 5' end; iii) a step for ligating said adenylated single strand anchor selectively to the 3' end of the cDNA strand of said cDNA/mRNA hybrid to select full-length cDNA/mRNA hybrid; and iv) a step for selectively amplifying a part of the full-length cDNA strand through a PCR reaction which employs a gene-specific primer (GSP) which base sequence is complementary to that of target genes.

Yet another object of the present invention is to provide a process for preparation of full-length cDNA in large scale through gene-cloning, which comprises: i) a step for preparing a hybrid composed of an mRNA strand and a cDNA strand of which three(3) or four(4) dCMPs are combined, incorporated or attached at the 3' end, by treating mRNA with reverse transcriptase; separately from the above step, ii) a step for adenylating a single strand anchor of which a biotin or phosphate group is combined, incorporated or attached at the 3' end and a phosphate group is combined, incorporated or attached at the 5' end; iii) a step for ligating said adenylated single strand anchor selectively to 3' end of the cDNA strand of said cDNA/mRNA hybrid to select full-length cDNA/mRNA hybrid; iv) a step for amplifying only the full-length cDNA strand through PCR which employs a primer of which base sequence is complementary to that of said anchor; v) a step for making double stranded cDNA which has specific cohesive ends, by cleaving the specific site of said anchor ligated on full-length cDNA prepared through the above step i) with restriction enzyme; vi) a step for inserting said double strand cDNA containing cohesive ends into a vector by using DNA ligase; vii) a step for transforming the vector which contains said cDNA into host cells; viii) a step for cloning said host cells in large scale; and ix) a step for separating the full-length cDNA from the vector obtained from said host cells, by cleaving full-length cDNA from said vector with DNA restriction enzyme which is used in step v).

Thus, the present invention provides a process for the preparation of full length cDNA which comprises steps i) to iv) as defined herein followed by cloning double stranded full length cDNA into an appropriate vector and expressing the vector in an appropriate host cell after which the full length cDNA can be isolated on a large scale. The double stranded cDNA with specific cohesive ends, vectors comprising the full length cDNA molecules, or host cells comprising said vectors, which cDNA, vectors or host cells are produced, obtained or obtainable by the methods of the invention as described herein form yet further aspects of the invention. In addition, processes which comprise steps i) to v), steps i) to vi), steps i) to vii), steps i) to viii) or steps i) to ix) outlined above form further aspects of the invention.

Appropriate PCR primers for use in the steps of the various processes described herein can readily be designed by a person skilled in the art. For example, where it is desired to specifically amplify the full length cDNA strand, appropriate primers which can interact with the 5' and 3' ends of the full length cDNA can readily be chosen. For example, in the methods of the present invention the amplification of full length cDNA ideally employs a primer which base sequence is complementary to that of the adenylated single strand anchor (e.g. a CapFishRace primer as described herein), and an oligo-dT primer (e.g. CapFish(dT) as described herein), which is complementary to the poly A sequence of the cDNA molecules generated and is located at the other end of the full length cDNA molecules to the anchor sequence.

Where it is desired to specifically amplify a part of a cDNA molecule, again appropriate primers which can interact with the two ends (the 5' and 3' ends) of the particular part it is wished to amplify can readily be designed. For example, in the methods of the present invention, the amplification of a part of the full length cDNA molecule generated by the methods of the invention ideally employ a primer which :.s complementary to a specific part of the specific target cDNA molecule in question which it is wished to amplify, i.e. is a gene-specific primer (GSP), together with a second primer which can interact with the other end of the part of the cDNA molecule it is wished to amplify, e.g. a primer which base sequence is complementary to that of the adenylated single strand anchor (e.g. a CapFishRace primer as described herein), or an oligo-dT primer (e.g. CapFish(dT) as described herein), which is complementary to the poly A sequence of the full length cDNA molecules generated.

Ideally the primers used will be fully complementary to the nucleic acid sequences with which they interact, i.e. all of the bases of the primer sequences will be complementary to the target sequence with which they interact. Some mismatch may however be tolerated and the main requirement is that the primers are sufficiently or substantially complementary to their target sequence so that they interact or hybridise or bind specifically with their target sequence. Thus the term "complementary" as used herein also includes such sufficiently or substantially complementary sequences.

Yet another object of the invention is to provide a process for obtaining full-length cDNA, which comprises:
i) a step for preparing a hybrid composed of an mRNA strand and a cDNA strand of which three(3) or four(4) dCMPs are combined, attached or incorporated at the 3' end by treating the mRNA with reverse transcriptase; separately from the above step,
ii) a step for adenylating a single strand anchor of which a biotin or phosphate group is combined at the 3' end and a phosphate group is combined at the 5' end; and
iii) a step for ligating selectively said single strand anchor to the 3' end of the cDNA strand of the full-length cDNA/mRNA hybrid to select full-length cDNA/mRNA hybrid.

Full length cDNA or a part thereof produced, obtained or obtainable by the methods of the invention as described herein form yet further aspects of the invention.

A preferred reverse transcriptase(RTase) used herein is M-MLV, which is a kind of terminal transferase capable of adding three(3) or four(4) dCMPs to the 3' end of the cDNA by recognizing the 5' cap structure of mRNA. More information about RTase is described in "Nucleic acids Research, 1999, vol, 27, No 21, e31" of Schmidt and Mueller in detail.

The exemplary RNA ligase used herein is T4 RNA ligase which can recognize trinucleosidediphosphate (NpNpNOH) as a minimum substrate for a phosphate acceptor and also recognize nucleoside 3',5'-biphosphate group(pNp) as a minimum substrate for a phosphate donor.

Therefore, only primary full-length cDNA containing three or more template-independent cytosine residues, can be ligated to other oligomers by T4 RNA ligase. However, cDNA containing two or less dNTPs at its 3' end, cannot be ligated to other oligomers since two or less dNTPs cannot be recognized by T4 RNA ligase.

That is, only the primary full-length cDNA containing three or more dCMPs at its 3' end, which was added through template-independent reaction by reverse transcriptase (Rtase), can be recognized by T4 RNA ligase during the primary cDNA synthesis.

Optionally, the processes of the present invention may further comprise an additional step between step iii) and step iv), or after step iii), for removing the residual mRNA which is not participating in cDNA synthesis, e.g. by using ribonuclease such as RNase A.

A single strand anchor of which a biotin or phosphate group is combined, attached or incorporated at the 3' end and a phosphate group is combined, attached or incorporated at the 5' end to be ligated with another single strand, is prepared in the process of the present invention; and the single strand anchor thus prepared is ligated to the 3' end of full-length cDNA wherein three(3) or four(4) cytosine residues are combined, attached or incorporated by treating with RNA ligase such as T4 RNA ligase; and the cDNA thus selected is amplified through PCR by using an anchor-specific primer.

Through the process of the present invention, double strand cDNA can be completely amplified by PCR wherein the above described anchor specific primer and CapFish(dT) primer are employed to produce cDNA library.

Thus, the use of cDNA molecules prepared, obtained or obtainable by the methods of the present invention in biological applications or methods, e.g. in the preparation of cDNA libraries or in other cloning techniques or the analysis of gene structure, expression or function is also provided.

It can be seen from the above discussion that a yet further aspect of the invention provides an anchor which contains one or more adenine residues or groups combined, incorporated or attached at its 5' end. Preferably such an anchor also contains one or more restriction sites and in particular one or more recognition sites recognizable by a restriction enzyme selected from the group consisting of *Not*I, *Sma*I, *Xba*I and *Xho*I. In addition such anchors preferably contain one or more regions with sequences that are complementary to a primer, e.g. a PCR primer, and preferably a primer employed for the amplification of full length cDNA in accordance with the present invention. A further preferred feature is that the anchors are single stranded. Said anchors may also contain a biotin or phosphate group at the 3' end and a phosphate group at the 5' end, see e.g. the CapFishLink primer of Table 1 which is preferred.

Primers which can specifically bind to such anchors are also provided. Said anchor-specific primers generally comprise one or more thymine groups and three to four guanine groups to enable them to bind to the anchors of the invention. Such thymine and guanine groups are generally positioned at the 3' end of the primers. Preferred primers are the CapFishRace primers shown in Table 1.

The time required for the process of the present invention is shorter than that of the conventional processes since the additional step for treatment of terminal transferase is not required for the process of the present invention.

More than eight(8) hours is required for ligation between the anchor and nucleic acid for the best yield of the process of the present invention. However, the pre-adenylated anchor which contains phosphate group, can be ligated with nucleic acid within three(3) hours. Consequently, the reaction time required for full-length cDNA selection which should be repeated for several times, can be shortened and thereby, the whole reaction time can be reduced.

The pre-adenylated anchor thus prepared, is so stable that it can be stored at room temperature for several weeks. Three(3) hours of ligation time is sufficient to ligate pre-adenylated anchor to nucleic acid with high yield, whereas more than eight(8) hours of ligation time is required for obtaining the sufficient amount of template strands sufficient to be amplified.

The process of the present invention is less complicated than "Capselect" method since the step for treatment of terminal transferase can be omitted.

### Industrial Applicability

The process of the present invention is a more efficient and simple process than conventional processes to prepare full-length cDNA since the process of the present invention requires only two steps for the preparation of full-length cDNA; one is a step for reverse transcription for synthesis of cDNA from mRNA and the other is a step for ligation between pre-adenylated anchor and the full-length cDNA. Consequently, the whole reaction time for selective amplification of full-length cDNA, can be shortened.

Gene cloning required to construct cDNA libraries, can be carried out more easily through the process of the present invention than conventional processes since there is a recognition site for specific restriction enzyme such as *Not*I, *Sma*I, *Xba*I, *Bgl*II, *Xho*I, *Sal*I in the Capfish primer of the present invention.

In addition, ligation between the anchor and full-length cDNA can be carried out more easily through the process of the present invention than conventional processes since the anchor is adenylated previously.

Therefore, full-length cDNA which can provide important information to reveal the structure of a gene and the function thereof, can be amplified more easily and efficiently through the process of the present invention than conventional processes.

Hereinafter, the present invention will be described in greater detail with reference to the following examples. The examples are given only for illustration of the present invention and not to be limiting the present invention.

### Example 1.

### Selection of full-length cDNA and amplification of 5' end region of the full-length cDNA

The cDNA of TFR (transferrin receptor) RNA which exists in small amount in cell, and the cDNA of β-actin mRNA and the cDNA of GAPDH mRNA which exist in large amount in cell, were selected from the cDNAs prepared through reverse transcription by using 2µg of total RNAs or 100 ng of total mRNA. Then, the cDNAs thus prepared were amplified through the following process of the present invention.

### 1-1: synthesis of cDNA strand

Total RNA was extracted from tissue of human placenta, spleen and liver by using Blood RNA PrepMate™(Bioneer, Korea). Messenger RNA was extracted by using dT celluose column, if needed.

2µg of total RNA or 100 ng of mRNA was used to synthesize cDNA strand by using oligo(dT) anchor primer[CapFish(dT)]. 20µl of the reaction mixture for reverse transcription, which contains 50mM Tris-HCl pH 8.3, 75mM KCl, 6 mM MgCl₂, 10 mM DTT, 1 mM dNTPs each and 1µl of PowerScript™ RTase (Clontech, USA), was incubated for one(1) hour at 42°C. This reaction mixture for reverse transcription was described in US. Patent No. 4,943,531 in detail. 0.4µl of 100 mM MnCl₂ was added to the reaction mixture, if needed. Then the reaction mixture was incubated for 30 min. at 42°C. To make reaction mixture, volume was 100µl, ddH₂O was added and then it was extracted by using phenolchloroform. Then, 10µl of 3M sodium acetate/DEPC and 200µl of absolute ethanol were added into the reaction mixture to precipitate RNA and cDNA/mRNA hybrid.

### 1-2: synthesis of anchor and ligation between the anchor and nucleic acid by T4 RNA ligase

300 pmole of the anchor (CapFishLink) was incubated with 50mM Tris-HCl pH 7.5, 10mM MgCl₂, 10mM DTT, 1mM ATP, 10µl of 25% PEG 6000, 10% DMSO, 0.006% BSA and 30 unit of T4 RNA ligase(Takara, Japan) for twelve(12) hours at 37°C without using RNA and cDNA/mRNA hybrid obtained in Example 1-1.

60 pmole of the anchor prepared in the above step, was added to the reaction mixture containing RNA and cDNA/mRNA hybrid obtained in Example 1-1. Then, the reaction mixture was treated with 10 unit of T4 RNA ligase for three(3) hours at 37°C. RNAs which had not been participated in cDNA synthesis, were removed by using 4 µl of 10 mg/ml RNase for 30 min. at 37°C.

### 1-3: amplification through PCR

1 µl of the reaction mixture prepared in Example 1-2, was used as a template to amplify 3' end region and 5' end region of β-actin mRNA through RACE(rapid amplification of cDNA end).

The anchor-specific primer (CapFishRaceN 1 and 2) and the anti-sense primer(β-ActinR) of β-actin mRNA(Genebank accession No: NM_001101), were used to amplify 5' end region of β-actin mRNA through 5'-end RACE. The CapFish(dT) and the sense-primer of β-actin mRNA (β-ActinF) were used to amplify 3' end region of β-actin mRNA through 3'-RACE. The condition for PCR such as the 3'-RACE and the 5'-RACE of the present invention, was as follows:

The polymerase chain reaction was initiated for 5 min. at 94°C, and was carried out for 1 min. at 94°C, for 2 min. at 60°C and then, for 3 min. at 72°C. The above steps were repeated five(5) times.

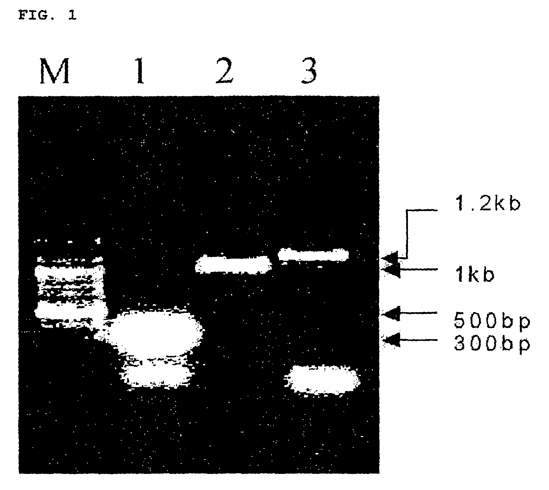

Then, supplemental reaction was carried out for 1 min. at 94°C, for 1 min. 58°C and for 2 min. at 72°C. Such supplementary reaction was repeated thirty(30) times. Then, final amplification reaction was carried out for 5 min. at 72°C. The result was represented in Fig 1.

In Fig. 1, M represents the size marker. Lane 1 represents the result of agarose gel-electrophoresis of the β-actin cDNA amplified through PCR which employs β-actin sense-primer and β-actin anti-sense primer. Lane 2 represents the result of agarose gel-electrophoresis of the 3' end region of β-actin cDNA amplified through 3'-RACE by using CapFish(dT) primer and β-actin sense-primer. Lane 3 represents the result of agarose gel-electrophoresis of the 5' end region of β-actin cDNA amplified through 5'-RACE by using CapFishRacel primer and β-actin anti-sense primer.

The primer for GAPDH gene(Genebank accession No: M33197), the primer for RNA polymerase II gene and the primer for TFR gene(Genebank accession No: X01060), together with CapFishRace1 primer, were employed for amplification of the 5' end region of each cDNA through 5'-RACE.

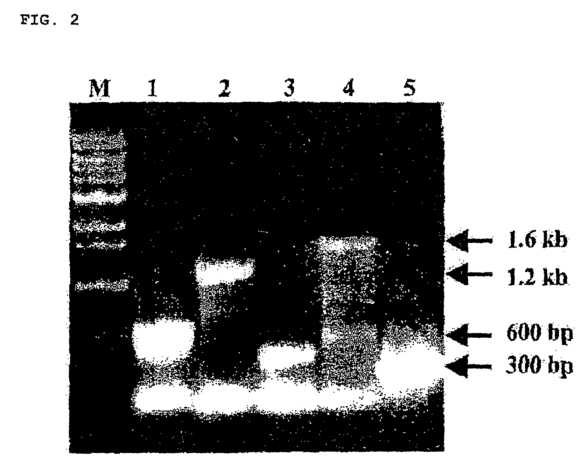

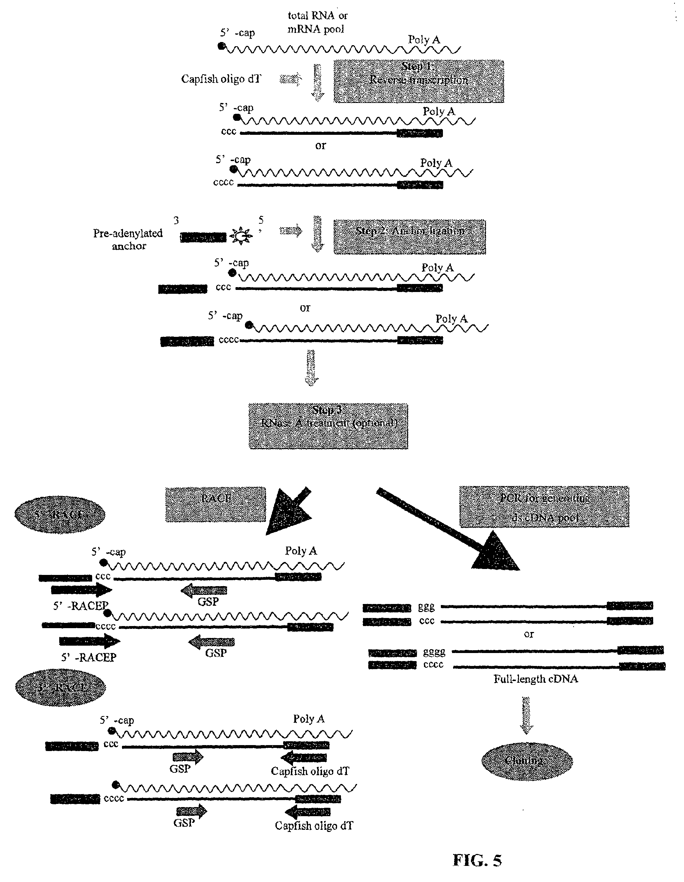

As represented in Fig. 2, 5' end region of GAPDH gene and β-actin gene which exist sufficiently in cell, and 5' end region of RNA polymerase and TFR gene which exist in medium or small amount in cell, could be amplified through one(1) round of 5'-RACE by using 100 ng of each mRNA.

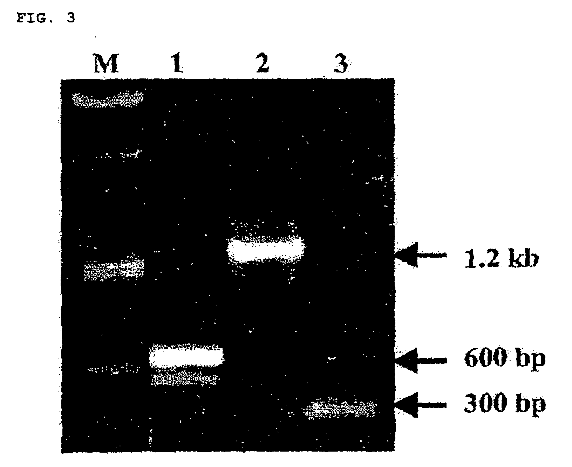

As represented in Fig 3, 5' end region of GAPDH, 5' end region of γ-actin and 5' end region of RNA polymerase could be amplified through one(1) round of 5'-RACE by using total RNA.

### 1-4: determination of sequence for product of reverse transcription

The cDNA amplified through PCR in which AccuPrep™ gel purification kit(Bioneer, Korea) was employed, was purified and cloned. Then, the reaction for determination of sequence of the cDNA was carried out by using AccuPrep™ DNA sequencing kit(Bioneer, Korea). Then, the resulting reaction mixture was applied on 4% denaturing gel and detected with Silverstar™ staining kit(Bioneer, Korea) to determine their base sequences.

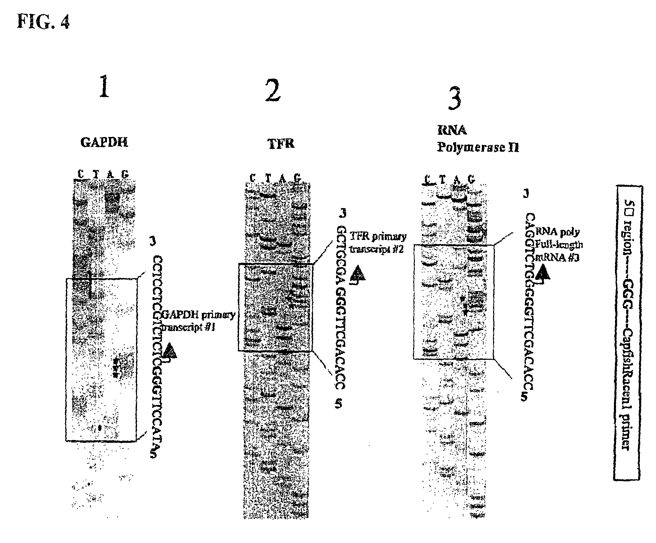

As represented in Fig. 4, the base sequences of 5' end region of GAPDH gene(Genebank accession No: J04038), 5' end region of RNA polymerase II gene(Genebank accession No: X984331.1) and 5' end region of TFR gene(Genebank accession No: X04664), were corresponded to their base sequences which had been reported previously.

The above results show the specificity of the process of the present invention, which is capable of amplifying mRNA existing in small amount in cell such as TFR gene in case that 100 ng of mRNA was used.

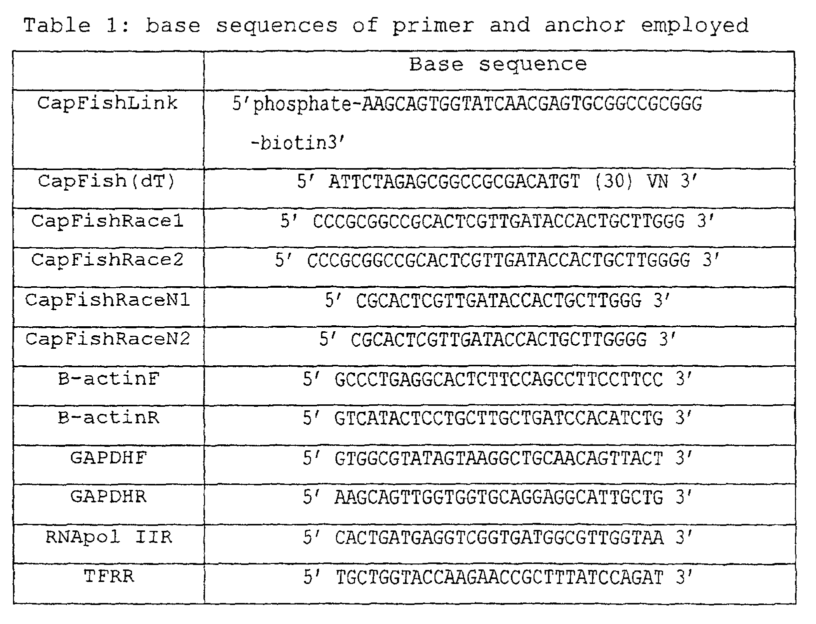

Fig.1 represents the result of agarose gel electrophoresis of the products obtained by amplification of both ends of β -actin cDNA by the process and the primer of the present invention.

Lane 1 is the result of amplification in which sense primer and anti-sense primer were employed. Lane 2 and Lane 3 are the result of amplification of 3' end region and 5' end region of β-actin cDNA respectively, in which the sense and anti-sense gene-specific primer and the anchor-specific primer were employed. The length of all products corresponds to the length derived from the full-length β-actin gene which had been reported previously.

Fig. 2 represents the result of agarose gel electrophoresis of the products obtained by amplification of 5' end region of full-length GAPDH(Lane 1), 5' end region of full-length β-actin(Lane 2), 5' end region of full-length of RNA polymerase II(Lane 3) and 5' end region of full-length TFR(Lane 4) through the process and the primer of the present invention by using 100 ng of mRNA. Lane 5 is the result of agarose gel electrophoresis of the product(300bp) of a portion of GAPDH gene, which was amplified by using sense and anti-sense primer.

Fig. 3 represents the result of agarose gel electrophoresis of the products obtained by the amplification of GAPDH(Lane 1), γ-actin(Lane 2), RNA polymerase II(Lane 3) prepared from 2µg of total spleen mRNA by using the process and the primer of the present invention.

Fig. 4 represents the result of sequence analysis in which the base sequences determined by amplifying 5' end of full-length GAPDH gene(Fig. 4-1), 5' end of full-length TFR gene(Fig. 4-2) and 5' end of full-length RNA polymerase II gene(Fig. 4-3) by using the process and the primer of the present invention, and then by cloning them, were compared with their full-length sequences which had been reported previously.

Fig. 5 represents the brief process of the present invention for the preparation of full-length cDNA.

## Claims

1. A process for selective amplification of full-length cDNA, which comprises:
i) a step for preparing a hybrid composed of an mRNA strand and a cDNA strand of which three(3) or four(4) dCMPs are combined at the 3' end, by treating the mRNA with reverse transcriptase;
separately from the above step,
ii) a step for adenylating a single strand anchor of which a biotin or phosphate group is combined at the 3' end and a phosphate group is combined at the 5' end;
iii) a step for ligating said adenylated single strand anchor selectively to the 3' end of the full-length cDNA strand of said cDNA/mRNA hybrid to select full-length cDNA/mRNA hybrid; and
iv) a step for amplifying only the full-length cDNA strand through a polymerase chain reaction which employs a primer of which base sequence is complementary to that of said anchor.

2. A process for selective amplification of a part of a cDNA or mRNA, which comprises:
i) a step for preparing a hybrid composed of an mRNA strand and a cDNA strand of which three(3) or four(4) dCMPs are combined at the 3' end, by treating the mRNA with reverse transcriptase;
separately from the above step,
ii) a step for adenylating a single strand anchor of which a biotin or phosphate group is combined at the 3' end and a phosphate group is combined at the 5' end;
iii) a step for ligating said adenylated single strand anchor selectively to the 3' end of the cDNA strand of said cDNA/mRNA hybrid to select full-length cDNA/mRNA hybrid; and
iv) a step for selectively amplifying a part of the full-length cDNA strand through a polymerase chain reaction which employs a gene-specific primer which base sequence is complementary with that of target genes.

3. A process for preparation of full-length cDNA, which comprises:
i) a step for preparing a hybrid composed of an mRNA strand and a cDNA strand of which three(3) or four(4) dCMPs are combined at the 3' end, by treating the mRNA with reverse transcriptase;
separately from the above step,
ii) a step for adenylating a single strand anchor of which a biotin or phosphate group is combined at the 3' end and a phosphate group is combined at the 5' end;
iii) a step for ligating said adenylated single strand anchor selectively to the 3' end of the cDNA strand of said cDNA/mRNA hybrid to select full-length cDNA/mRNA hybrid;
iv) a step for amplifying only the full-length cDNA strand through a polymerase chain reaction which employs a primer of which base sequence is complementary with that of said anchor;
v) a step for making double strand cDNA which has specific cohesive ends, by cleaving the specific site of said anchor ligated on full-length cDNA prepared through the above step i) to step iv), with a restriction enzyme;
vi) a step for inserting said double strand cDNA containing cohesive ends into a vector by using DNA ligase;
vii) a step for transforming the vector which contains said cDNA into host cells;
viii) a step for cloning said host cells in large scale; and
ix) a step for separating the full-length cDNA from the vector obtained from said host cells, by cleaving full-length cDNA from said vector with the restriction enzyme which is used in step v).

4. The process according to any one of claims 1 to 3, wherein said reverse transcriptase is M-MLV.

5. The process according to any one of claims 1 to 3, wherein said RNA ligase is T4 RNA ligase.

6. The process according to any one of claims 1 to 3, which further comprises between step iii) and step iv) :
a step for removing the residual single strand mRNA by using ribonuclease.

7. The process according to claim 6, wherein said ribonuclease is RNase A.

8. The process according to any one of claims 1 to 3, which further comprises a step for ligation of adenine group to said anchor.

9. An anchor which contains one or more adenine groups combined at its 5' end.

10. The anchor according to claim 9, wherein said anchor contains a recognition site recognizable by a restriction enzyme selected from the group consisting of *Not*I, *Sma*I, *Xba*I and *Xho*I.

11. The anchor according to claim 9 or claim 10, wherein said anchor contains one or more regions of which base sequence is complementary to that of a primer.

12. The anchor according to any one of claims 9 to 11, wherein said anchor is single stranded.

13. The anchor according to any one of claims 9 to 12, wherein said anchor contains one or more regions of which base sequence is complementary to that of primer employed for amplification of cDNA.

14. A primer which specifically binds to an anchor containing one or more thymine group and three(3) to four(4) guanine groups combined at its 5' end.

15. A process for obtaining full-length cDNA, which comprises:
i) a step for preparing a hybrid composed of an mRNA strand and a cDNA strand of which three(3) or four(4) dCMPs are combined at the 3' end by treating the mRNA with reverse transcriptase;
separately from the above step,
ii) a step for adenylating a single strand anchor of which a biotin or phosphate group is combined at the 3' end and a phosphate group is combined at the 5' end; and
iii) a step for ligating selectively said single strand anchor to the 3' end of the cDNA strand of the full-length cDNA/mRNA hybrid to select full-length cDNA/mRNA hybrid.

16. The process according to claim 15 wherein the reagents used are as defined in any one of claims 4 to 5.

17. The process according to claims 15 and 16 comprising a further step in accordance with any one of claims 6 to 8.

18. Full length cDNA or a part thereof obtainable by the processes as defined in any one of claims 1 to 8 or claims 15 to 17.
